Europäisches Patentamt

European Patent Office

Office européen des brevets

(19)

(11) Publication number: **0 451 927 A1**

## EUROPEAN PATENT APPLICATION

(21) Application number: 91201451.1

(51) Int. Cl.5: **C07K 5/12**

(22) Date of filing: **18.11.83**

This application was filed on 12 - 06 - 1991 as a divisional application to the application mentioned under INID code 60.

(30) Priority: **22.11.82 US 443513**
**22.11.82 US 443763**
**22.11.82 US 443764**

(43) Date of publication of application:
**16.10.91 Bulletin 91/42**

(60) Publication number of the earlier application in accordance with Art.76 EPC: **0 109 681**

(84) Designated Contracting States:
**AT BE CH DE FR GB IT LI LU NL SE**

(71) Applicant: **E.I. DU PONT DE NEMOURS AND COMPANY**
**1007 Market Street**
**Wilmington Delaware 19898(US)**

(72) Inventor: **Stoutamire, Donald W.**
**904 Bel Passi Drive**
**Modesto, California 95350(US)**
Inventor: **Tieman, Charles H.**
**2209 Fremont Street**
**Modesto, California 95350(US)**
Inventor: **Dong, Walter**
**14628 River Forest**
**Houston, Texas 77079(US)**

(74) Representative: **Jones, Alan John et al**
**CARPMAELS & RANSFORD 43 Bloomsbury Square**
**London, WC1A 2RA(GB)**

(54) Cyclo(D-phenylalanyl-D-histidine).

(57) Cyclo(D-phenylalanyl-D-histidine) can be used as a catalyst in the preparation of an optically-active, optionally substituted S-alpha-cyano-3-phenoxy benzyl alcohol.

EP 0 451 927 A1

Rank Xerox (UK) Business Services

EP 0 451 927 A1

This invention relates to a catalyst for use in the preparation of an optically-active, optionally substituted S-alpha-cyano-3-phenoxybenzyl alcohol or a mixture enriched therein.

The catalyst of the invention is an optically-active histidine-containing cyclic peptide, namely cyclo(D-phenylalanyl-D-histidine). The catalyst is prepared by conventional peptide synthesis, for example, as in Greenstein, J.P. and M. Winitz, "Chemistry of the Amino Acids", John Wiley & Sons, Inc., New York, 1961.

The asymmetric carbons of the catalyst have the D-configuration.

Functional groups in the dipeptide catalyst can contain protecting groups; any conventional amino acid protecting group known in the art can be used. For example, the protecting group is an organic acid in the case of the free N-H. Any organic acid which will not interfere with the reaction can be used as the protecting group. Preferably, the protecting group is another amino acid. Any amino acid can be used, but, preferably, the amino acid is non-heterocyclic and is a monoamino or diamino-alkanoic or aralkanoic acid, such as alanine, phenylalanine, glutamic acid, glycine and the like.

To make an optically-active optionally substituted S-alpha-cyano-3-phenoxybenzyl alcohol an optionally substituted 3-phenoxybenzaldehyde is treated with a source of hydrogen cyanide in the presence of a substantially water-immiscible, aprotic solvent and in the presence of a cyclo(D-phenyl-alanyl-D-histidine) dipeptide catalyst.

This process for making an optically-active, optionally substituted S-alpha-cyano-3-phenoxybenzyl is described and claimed in European patent application 83111562.1 (Publication No. 0109681) of which this application is a divisional.

When the catalyst is prepared by a conventional method in the presence of solvent (e.g. water), it can, if solid, also contain solvent (e.g. water) of crystallisation. The optically-active cyclo-(D-phenylalanyl-D-histidine) dipeptide catalyst of the invention thus may include the presence or absence of solvent (e.g. water) of crystallisation when solid.

Preparations

Preparation 1 - N-(Benzyloxycarbonyl)-D-phenylalanine

A 15.0 sample of D-phenylalanine was dissolved in 45 ml of aqueous solution containing 7.26 g of 50% sodium hydroxide. This solution was stirred at 0-10°C as 16.3 g of benzyl chloroformate was added rapidly in portions. The resulting reaction was mildly exothermic, and shortly after addition, solids precipitated. An additional 45 ml of water and 3.63 g of 50% sodium hydroxide were added, causing most of the solids to redissolve. The reaction mixture was stirred for 20 minutes and then acidified with 6 N hydrochloric acid. The resulting solids were filtered, washed with water and then with hexane, and dried by suction and then under vacuum to give 47 g of white solids. These solids dissolved in ether were washed twice with 1 N hydrochloric acid and then with water, dried over $MgSO_4$ and stripped to 35°C at 2.5 mm Hg to give 27.7 g of the desired product as a colourless oil.

Preparation 2 - N-(Benzyloxycarbonyl)-D-phenylalanine, p-nitrophenyl Ester

A 300 ml three-neck flask with stirrer and dropping funnel was charged under a nitrogen atmosphere with 27 g of the acid of Preparation 1 above in 135 ml of pyridine, followed by 13.2 g of p-nitrophenol. The resulting solution was cooled to 0° to 10°C as 14.6 g of phosphorus oxychloride was added. The resulting mixture was warmed to 25°C, stirred for 15 minutes, then poured into 300 ml of ice water. Filtration of the resulting solid, followed by washing with water and drying by suction, gave 33 g of product. This was crystallized from 340 ml of hot ethyl alcohol with chilling to -5°C. The product was filtered, washed with chilled ethyl alcohol, then with hexane, and sucked dry to give 28.7 g of the desired product, m.p. 122.5-124.5°C, $[\alpha]_D^{23}$ +24.7 (c 2.0, dimethylformamide).

Preparation 3- N-(Benzyloxycarbonyl)-D-phenylalanyl-D-histidine Methyl Ester

To a stirred solution of 5.0 g of D-histidine methyl ester hydrochloride in 40 ml of methylene chloride was added 4.18 g of triethylamine followed by 8.27 g of the nitrophenyl ester prepared as in Preparation 2 above. The reaction mixture immediately became bright yellow and solids began to precipitate. The reaction mixture was stirred for 2 hours, then stored overnight at -10°C. The reaction mixture was rewarmed to room temperature, and 0.6 ml of triethylamine was added. Then, 490 mg of the D-histidine methyl ester hydrochloride was added, and stirring was continued for 2 hours. The reaction mixture was washed with 20 ml of water, then twice with 20 ml of 10% ammonium hydroxide, and then twice with 20 ml of water. All the

2

washes were back-extracted serially with 20 ml of methylene chloride, and the combined organic phases was dried with MgSO₄ and stripped to 100 ml, filtered through silica, followed by 25 ml of 20% methanol in ethyl acetate. The resulting eluate was stripped to 40 ml and diluted to 120 ml with diethyl ether; the precipitated solid was filtered, washed with diethyl ether, and dried by suction to give 5.66 g of the desired product as a white solid, m.p. 114.5-117° C $[\alpha]_D^{20}$ -55.5 (c 2 in $CHCl_3$).

Example 1 - Cyclo(D-phenylalanyl-D-histidine)

5.60 g of methyl ester of Preparation 3 above was stirred and hydrogenated in 100 ml of methanol over 220 mg of 10% palladium on carbon at atmospheric pressure. After 3 hours, solids began to precipitate; an additional 2.5 ml of methanol was added to facilitate stirring. After 7 hours, an additional 280 ml of methanol was added as the mixture was heated to reflux. The mixture was filtered hot, and the filtrate was stripped to a gel-mush, which was mixed with 100 ml of diethyl ether. The resulting solid was filtered, washed with diethyl ether, and dried by suction and then under high vacuum at 35° C to give 3.29 g of the desired product as an off-white powder, $[\alpha]_D^{23} = +68.5$ (c 2.0 in $CH_3COOH$).

Example 2 - S-alpha-Cyano-3-phenoxybenzyl Alcohol

A 100 ml three-neck Bantam-ware flask was charged with 43 mg of cyclo(D-phenylalanyl-D-histidine) and put under a nitrogen atmosphere. Then, 3.51 ml of hydrogen cyanide was added by syringe causing the catalyst to swell and become a gel. After 5 minutes, 30 ml of toluene was added, causing additional catalyst to precipitate. 5.95 g of 3-phenoxybenzaldehyde was added all at once. The reaction mixture was stirred for 4.75 hours and then quenched with 20 ml of water containing 10 drops of concentrated hydrochloric acid. The toluene solution was separated, washed twice with water, and diluted to 50 ml with toluene for analysis, which showed 80% S-alpha-cyano-3-phenoxybenzyl alcohol isomer was produced.

Example 3 - S-alpha-Cyano-3-phenoxybenzyl Alcohol

The reaction of Example 2 above was repeated using 171 mg of cyclo(D-phenylalanyl-D-histidine). At intervals, 0.25 ml samples were removed and examined by gas liquid chromatography as follows:

| Time | % Conversion of Aldehyde |
|---|---|
| 35 minutes | 20 |
| 2 hours | 76 |
| 6.5 hours | 95 |

After 7 hours, the reaction mixture was quenched by addition of 10 ml of 1 N hydrochloric acid. The organic phase was separated and washed twice with water, dried over MgSO₄, filtered and stored at -10° C. The filtrate was diluted to 50 ml with toluene and the optical rotation was determined to be -1.54° at 21° in 1 dm cell. A sample of the product was acetylated with p-nitrophenylacetic anhydride and the stereoisomer ratio was determined by HPLC on a chiral Pirkle column to be 71% S-alpha-cyano-3-phenoxybenzyl alcohol and 29% R-alpha-cyano-3-phenoxybenzyl alcohol.

Example 4 - S-alpha-Cyano-3-phenoxybenzyl Alcohol

Two small round-bottom flasks having magnetic stirrers and septum covers were each charged with 22.5 mg of cyclo(D-phenylalanyl-D-histidine) and put under nitrogen. A sample of 0.98 ml of hydrogen cyanide was diluted to 25 ml with toluene, and 5 ml of the solution was added via syringe to each flask. After about 5 minutes, 0.87 ml of 3-phenoxybenzaldehyde (POAL) was added to each flask. Flask No. 1 was stirred in an oil bath at 35° C and flask No. 2 was stirred in a water bath at 24-26° C. The results of these experiments are below.

|  | Flask No. 1 | | | Flask No. 2 | | |
| Time, hr | POAL, % | α-Hydroxy-nitrile, % | R/S | POAL, % | α-Hydroxy-nitrile, % | R/S |
|---|---|---|---|---|---|---|
| 0.5 | 19 | 81 | 8.7/91.3 | 18 | 82 | 15.8/84.2 |
| 1 | 12 | 88 | - | 13 | 87 | 14.4/85.6 |
| 2 | 12 | 88 | - | 12 | 88 | - |
| 4 | 10 | 90 | 11.4/88.6 | 12 | 88 | 19 0/81.0 |
| 8 | 10 | 90 | - | 13 | 87 | - |

Example 5 - S-alpha-Cyano-3-phenoxybenzyl Alcohol

A reaction wag conducted by contacting 0.0099 m/kg cyclo(D-phenylalanyl-D-histidine) with 0.99 m/kg of 3 phenoxybenzaldehyde followed by 2 2 m/kg of hydrogen cyanide and 190 ppm water. The reaction was conducted in toluene at 25°C. The product obtained with 93% conversion of aldehyde was 88% S-alpha-cyano-3-phenoxybenzyl alcohol isomer.

Example 6 - S-alpha-Cyano-3-phenoxybenzyl Alcohol

To 0.2933 g of cyclo(D-phenylalanyl-D-histidine) in 15 ml of diethyl ether at 25°C was added 2.907 g of 3-phenoxybenzaldehyde followed by 0.940 g of hydrogen cyanide. After 2 hours, 94% of the 3-phenoxybenzaldehyde had been converted and the product had an S-alpha-cyano-3-phenoxybenzyl alcohol enantiomeric excess of 84%. After 4 to 20 hours, 99.4% of the 3 phenoxybenzaldehyde had been converted and the product had an S-alpha-cyano-3-phenoxybenzyl alcohol enantiomeric excess of

Example 7 - S-alpha-Cyano-3-phenoxybenzyl Alcohol

A reaction was conducted by treating 0.0200 g of cyclo(D-phenylalanyl-D-histidine) at 25°C under nitrogen with 1.4037 g of 3-phenoxybenzaldehyde to disperse the catalyst followed by injecting 2.1812 g of toluene with 13.65 w% hydrogen cyanide. After 2.7 hours, 93% of the 3-phenoxybenzaldehyde was converted and the product had an S-alpha-cyano-3-phenorybenzyl alcohol enantiomeric excess of 77%.

Example 8 - S-alpha-Cyano-3-phenoxybenzyl Alcohol

A reaction was conducted at 25°C by contacting 0.0519 g of cyclo(D-phenylalanyl-D-histidine) in 9.32 ml of diethyl ether with 1.811 g of 3-phenoxybenzaldehyde followed by 0.617 g of hydrogen cyanide. After 3.6 hours, 99.4% of the 3-phenoxybenzaldehyde had been converted and the product had an S-alpha-cyano-3-phenoxybenzyl alcohol enantiomeric excess of 72%.

**Claims**

1. Cyclo(D-phenylalanyl-D-histidine).

2. Cyclo(D-phenylalanyl-D-histidine) protected by any organic acid which will not interfere with the reaction.

3. Cyclo(D-phenylalanyl-D-histidine) protected by another amino acid.

4. protected by alanine, phenylalanine, glutamic acid or glycine.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | J.C.S. CHEM. COMM., 1981, pages 229-230; J.-I. OKU et al.: "Asymmetric cyanohydrin synthesis catalysed by a synthetic cyclic dipeptide" * Entire document * | 1-4 | C 07 K 5/12 |
| P,X | WPIL/DERWENT, AN = 83-31127K [13], Derwent Publications Ltd, London, GB; & JP-A³58 029 757 (NIPPON KAYAKU K.K.) * Entire abstract * | 1 | |
| E | EP-A-0 132 392 (ICI AUSTRALIA LTD) * Entire document * | 1-4 | |

|  | TECHNICAL FIELDS SEARCHED (Int. Cl.5) |
|---|---|
|  | C 07 K |

The present search report has been drawn up for all claims

| Place of search | Date of completion of search | Examiner |
|---|---|---|
| The Hague | 11 July 91 | GROENENDIJK M.S.M. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document